# EUROPEAN PATENT APPLICATION

(11) **EP 3 885 769 A1**
(43) Date of publication of application: **29.09.2021**
(21) Application number: 20164987.8
(22) Date of filing: 23.03.2020
(51) Int. Cl.: G01N 33/68

(54) **METHODS FOR DIAGNOSING IMMEDIATE HYPERSENSITIVY REACTION**

(71) Applicant: Universiteit Antwerpen, 2000 Antwerpen (BE)
(72) Inventor: Ebo, Didier, 9150 Bazel (BE); Sabato, Vito, 2550 Kontich (BE); Mertens, Christel, 2100 Deurne (BE); Elst, Jessy, 2920 Kalmthout (BE)
(74) Representative: De Clercq & Partners

(57) **Abstract**

Provided herein are *in vitro* methods for diagnosing an immediate hypersensitivity reaction (IHR) against a small compound or a drug in a subject, comprising the steps of (a) contacting mast cells with serum obtained from said subject; (b) contacting the mast cells obtained in step (a) with said small compound or said drug; (c) detecting mast cell activation and/or degranulation of the mast cells obtained in step (b); and (d) attributing the detection or no detection of mast cell activation and/or degranulation to a particular diagnosis of IHR.

## Description

### FIELD

The present invention relates to methods for diagnosing immediate hypersensitivity reaction (IHR) in a subject.

### BACKGROUND

Immediate hypersensitivity reactions (IHRs) against drugs (i.e. medicinal products) constitute a significant and increasing health burden with potentially dramatic consequences of diagnostic error. However, correct diagnosis of IHRs is not always straightforward for many reasons. For example, the gold standard for the diagnosis of IHRs is a controlled graded drug challenge (DC), in which increasing doses of a drug or placebo are administered under strict medical supervision. Unfortunately, DC are hindered by different ethical (risk for anaphylaxis and fatalities) and practical (costly, time consuming) limitations that have hindered its entrance in mainstream practise. Moreover, full-dose DC might simply be impossible (e.g. anaesthetics and curarizing neuromuscular blocking agents (NMBAs)) or not be predictive for the clinical outcome. Therefore, in clinical practice, confirmatory testing of IHRs generally starts with skin tests or *in vitro* tests such as quantification of drug-specific IgE (sIgE) antibodies. However, skin testing is still associated with many uncertainties, especially for nonspecific histamine releasers that might act via off-target MRGPRX2 occupation (e.g. opiates and quinolones), whilst the few available drug-sIgE assays exhibit highly varying accuracy depending on the drug and clinical phenotype. Consequently, many efforts have been undertaken to improve diagnosis of IHRs against drugs. One of the strategies to develop tests that are more accurate has focused on *in vitro* activation of basophils (BAT). In the BAT, allergen-specific activation of patients' basophils is measured via flow cytometric analysis of the upregulation of specific surface markers such as CD63 and CD203c. The principles and utility of the BAT to diagnose IHRs against drugs have been assessed in multiple studies and was recently reviewed elsewhere. Overall, the BAT appears a promising diagnostic for IDRs, especially from NMBAs and some β-lactam antibiotics. The key strength of the BAT is that it does not require coupling of drugs to a solid phase. Coupling that might sometimes difficult and mask relevant epitopes. The major weaknesses of the BAT are the requirement for fresh patients' blood and the unpredictable basophilic non-responder status that is observed in about 5-15% of the population.

Therefore, there remains a pressing need for novel, safe and reliable techniques to study and correctly diagnose IHR to reduce the mortality risk.

### SUMMARY

The present inventors have addressed the challenges for the diagnosis of IHR to a drug by establishing that such reactivity can be established with high analytical sensitivity by mast cell activation tests (MATs). In MATs, mast cells (e.g. primary mast cells, mast cell lines, or cells with similar functionality, i.e. capable of activation and/or degranulation upon exposure to a small compound or drug) are contacted with patients' sera prior to exposing said mast cells to the small compound or drug whereby activation of the mast cells is indicative of hypersensitivity. First, it was unexpected that the sensitivity and the reliability of MATs are sufficiently high for diagnosing IHR to a drug. In addition, small compounds in general are considered to be less potent effector cell activators than larger compounds, particularly proteinaceous allergens such as food proteins, pollen or venoms. In many cases, titres of small compound-specific IgE or drug-specific IgE are rather low.

Advantageously, the MAT technique only makes use of the serum of the patient and not fresh, viable blood cells. Accordingly, the MAT circumvents the limitation of deterioration of blood cells in the blood sample and hence, the sample obtained from the patient does not need to be a fresh sample. Accordingly, the method of diagnosis of the present invention can be performed at a central diagnostic laboratory and can be repeated if part of the patient's serum is stored adequately. Furthermore, the present inventors established that the method of present invention correlates with clinical reactivity and can be applied to discriminate between IHR and merely sensitization, as, for example, reflected by a positive small compound-specific IgE result or drug-specific IgE result. Besides, the inventors' method demonstrates high analytical sensitivity depicting low titers of small compound-specific IgE or drug-specific IgE.

The invention provides *in vitro* methods for diagnosing an immediate hypersensitivity reaction (IHR) against a drug in a subject. The invention also provides methods for diagnosing an immediate hypersensitivity reaction (IHR) against a small compound in a subject; the methods of the invention comprise the steps of
- (a) contacting mast cells with serum obtained from said subject;
- (b) contacting the mast cells obtained in step (a) with said small compound or said drug;
- (c) detecting mast cell activation and/or degranulation of the mast cells obtained in step (b);
- (d) attributing the detection or no detection of mast cell activation and/or degranulation to a particular diagnosis of IHR.

In particular embodiments, said method is a method for diagnosing IHR against a small compound and step (b) comprises contacting the mast cells obtained in step (a) with said small compound. In particular embodiments the small compound has a molecular weight of at most 9000 Da, preferably at most 1000 Da. In particular embodiments, said small compound is a diagnostic or a therapeutic compound. In particular embodiments, said method is a method for diagnosing IHR against a drug and step (b) comprises contacting the mast cells obtained in step (a) with said drug.

In particular embodiments, in step (b) of the methods as taught herein, the mast cells are contacted with a non-toxic concentration of said small compound or said drug.

In particular embodiments, said mast cells used in step (a) are cultured donor mast cells (dMCs).

In particular embodiments, said dMCs used in step (a) are obtained by
- isolating peripheral blood mononuclear cells from peripheral blood obtained from a healthy subject;
- isolating CD34+ progenitor cells from said peripheral blood mononuclear cells; and
- culturing said CD34+ positive cells during a period from 4 to 12 weeks.

In particular embodiments, mast cell activation and/or degranulation in step (c) is detected by detecting one or more biomarkers of mast activation and/or degranulation in or on said mast cells or secreted by said mast cells.

In particular embodiments, mast cell degranulation in step (c) of the method as taught herein is detected by determining overexpression of the CD63 antigen on the cell surface of said mast cells.

In particular embodiments, the method as taught herein further comprises a step of pre-incubating said mast cells as obtained in step (a) with one or more priming cytokines selected from the group consisting of IL-33, IL-6 and IL-4, prior to step (b).

### BRIEF DESCRIPTION OF DRAWINGS

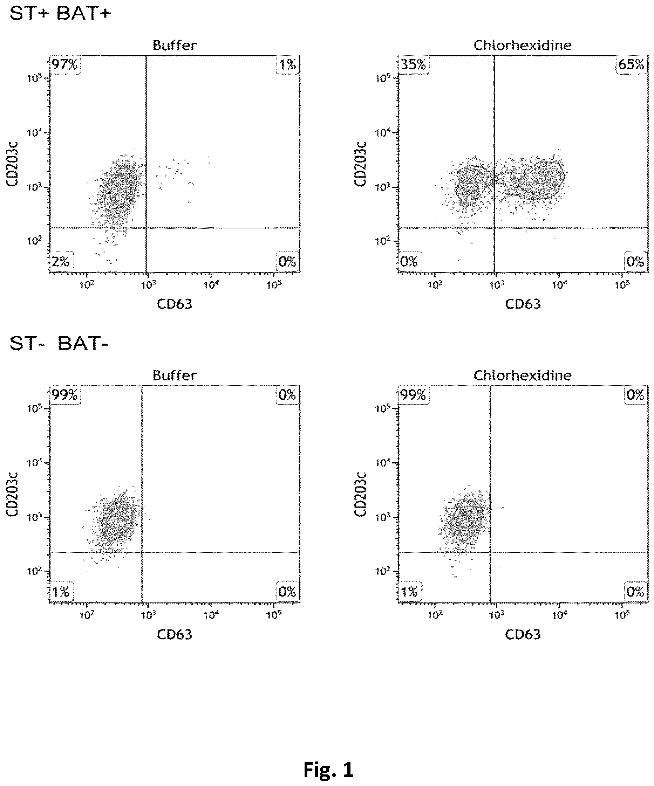

**Figure 1****. Representative plot of mast cell activation test with chlorhexidine.** Cultured human donor mast cells were activated with chlorhexidine (50000 ng/ml) after passive sensitization of the cells with a serum of a patient in whom diagnosis was unequivocally documented by positive skin test and basophil activation test (ST+BAT+) and a sensitized individual in whom diagnosis was discarded by negative skin test and basophil activation (ST-BAT-).

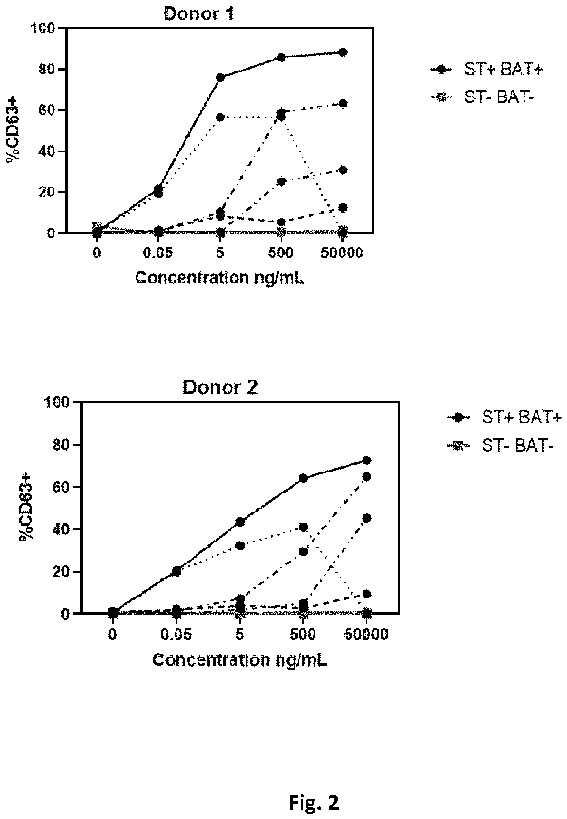

**Figure 2****. Mast cell activation with chlorhexidine.** Cultured human donor mast cells (dMC) (dMCs obtained from donor 1 (upper panel) and dMCs obtained from donor 2 (lower panel)) were activated with different concentrations of chlorhexidine (0.05, 5, 500 or 50000 ng/ml) after passive sensitization of the cells with a sera of patients with positive skin test and basophil activation test (ST+BAT+) (round symbols) or sensitized individuals with negative skin test and basophil activation (ST-BAT-) (square symbols). n =5, in each group

### DESCRIPTION

As used herein, the singular forms "a", "an", and "the" include both singular and plural referents unless the context clearly dictates otherwise.

The terms "comprising", "comprises" and "comprised of' as used herein are synonymous with "including", "includes" or "containing", "contains", and are inclusive or open-ended and do not exclude additional, non-recited members, elements or method steps. The terms also encompass "consisting of' and "consisting essentially of', which enjoy well-established meanings in patent terminology.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the invention herein is included to explain the context of the invention. This is not to be taken as an admission that any of the material referred to was published, known, or part of the common general knowledge in any country as of the priority date of any of the claims.

Throughout this disclosure, various publications, patents and published patent specifications are referenced by an identifying citation. All documents cited in the present specification are hereby incorporated by reference in their entirety. In particular, the teachings or sections of such documents herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the invention. When specific terms are defined in connection with a particular aspect of the invention or a particular embodiment of the invention, such connotation is meant to apply throughout this specification, i.e., also in the context of other aspects or embodiments of the invention, unless otherwise defined.

In the following passages, different aspects or embodiments of the invention are defined in more detail. Each aspect or embodiment so defined may be combined with any other aspect(s) or embodiment(s) unless clearly indicated to the contrary. In particular, any feature indicated as being preferred or advantageous may be combined with any other feature or features indicated as being preferred or advantageous.

Reference throughout this specification to "one embodiment", "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

Immediate hypersensitivity reactions (IHRs) to compounds such as diagnostic or therapeutic compounds as well as small compounds present in certain pharmaceutical compositions or food compositions, for example as adjuvants constitute a significant health issue with serious consequences of diagnostic error. Unfortunately, correct diagnosis of IHRs can pose a significant challenge, mainly because of knowledge gaps in the underlying mechanistic processes and the unavailability of or the limitations associated with currently available diagnostics. The present inventors have established a novel and reliable method to diagnose IHR to drugs as well as surprisingly to small compounds in general, with a high global accuracy and sensitivity. Indeed, present inventors established that such reactivity can be determined with high analytical sensitivity by mast cell activation tests (MATs) using the patients' sera. This surprising sensitivity allows the application of this testing for determining hypersensitivity to drugs. Moreover, this sensitivity for the detection of hypersensitivity to small compounds in general was unexpected, as small compounds are considered to be less potent effector cell activators than larger compounds, such as proteinaceous allergens such as food proteins, pollen or venoms. In many cases, titres of small compound-specific IgE or drug-specific IgE are rather low.

As the methods as taught herein do not require autologous cells from the subject, they do not require fresh viable blood samples. As a result, in contrast to the widely used basophil activation test (BAT), the methods as taught herein can be performed in a central diagnostic laboratory and can be repeated if part of the patient's serum is stored adequately. Furthermore, BAT requires basophils obtained from the subject. However, a non-responder status of the basophils to the BAT is observed in about 5-15% of the population. Hence, BAT is lost as a diagnostic in said cases as the results are not interpretable.

Furthermore, the methods as taught herein allow discriminating between genuine IgE/FcεRI-dependent allergy and mere sensitization, as reflected by a positive sIgE test result (i.e. for small compounds or drugs for which sIgE tests are available).

A first aspect provides the use of *in vitro* mast cell activation and/or degranulation as a biomarker for an immediate hypersensitivity reaction (IHR) against a drug or generally against a small compound in a subject.

The term "biomarker" is widespread in the art and may broadly denote a biological process or a biological molecule or a detectable portion thereof whose qualitative and/or quantitative evaluation in a subject is predictive or informative (e.g., predictive, diagnostic and/or prognostic) with respect to one or more aspects of the subject's phenotype and/or genotype, such as, for example, with respect to the status of the subject as to a given disease or condition. The present invention demonstrates that the qualitative and/or quantitative detection of activation and/or degranulation of mast cells (or cells with a similar functionality) by serum of a subject, can be used as a biomarker to assess immediate hypersensitivity of the patient to a drug and to a small compound in general.

The invention provides *in vitro* methods for diagnosing an immediate hypersensitivity reaction (IHR) against both drugs and generally against a small compound in a subject and/or for predicting the risk of anaphylaxis upon re-exposure of a small compound or a drug to a subject. The methods of the invention comprise the steps of
(a) contacting mast cells with serum obtained from said subject;
(b) contacting the mast cells obtained in step (a) with said small compound or said drug;
(c) detecting mast cell activation and/or degranulation of the mast cells obtained in step (b); and
(d) attributing the detection or no detection of mast cell activation and/or degranulation to a particular diagnosis of IHR against said small compound or said drug and/or to a particular prediction of anaphylaxis upon re-exposure of said small compound or said drug to a subject.

The term "immediate hypersensitivity reaction" or "IHR" as used herein refers to an excessive or inappropriate activation of the immune system triggered by the exposure of a substance, such as a small compound or a drug, wherein the excessive or inappropriate activation of the immune system occurs within a short period, preferably within 0 to 6 hours; more preferably within 0 to 120 minutes, after exposure to said substance. The IHR typically encompasses immunoglobulin E (IgE)-mediated release of histamine and other mediators from mast cells and basophils of the subject. IHR may manifest as clinical urticaria, vomiting, wheezing, angioma, bronchospasm and/or anaphylaxis. For example, anaphylaxis is the most severe IHR and typically occurs within minutes of exposure to the small compound or drug and can cause itchy rash, throat or tongue swelling, shortness of breath, vomiting, lightheadedness and low blood pressure and eventually death.

The terms "predicting" or "prediction", and "diagnosing" or "diagnosis" are commonplace and well understood in medical and clinical practice. By means of further explanation and without limitation, the term "diagnosing" as used herein refers to the process or act of recognising, deciding on or concluding on a disease or condition in a subject on the basis of symptoms and signs and/or from results of various diagnostic procedures (such as, for example, from knowing the presence, absence and/or quantity of one or more biomarkers characteristic of the diagnosed disease or condition). As used herein, "diagnosis of' the disease or condition as taught herein in a subject may particularly mean that the subject has such, hence, is diagnosed as having such. "Diagnosis of no" diseases or conditions as taught herein in a subject may particularly mean that the subject does not have such, hence, is diagnosed as not having such. A subject may be diagnosed as not having such despite displaying one or more conventional symptoms or signs reminiscent of such.

The term "predicting" or "prediction" generally refer to an advance declaration, indication or foretelling of a disease or condition in a subject not (yet) having said disease or condition. For example, a prediction of a disease or condition in a subject may indicate a probability, chance or risk that the subject will develop said disease or condition, for example within a certain time period or by a certain age. Said probability, chance or risk may be indicated *inter alia* as an absolute value, range or statistics, or may be indicated relative to a suitable control subject or subject population (such as, *e.g.,* relative to a general, normal or healthy subject or subject population). Hence, the probability, chance or risk that a subject will develop a disease or condition may be advantageously indicated as increased or decreased, or as fold-increased or fold-decreased relative to a suitable control subject or subject population. As used herein, the term "prediction" of the conditions or diseases as taught herein in a subject may also particularly mean that the subject has a "positive" prediction of such, *i.e.,* that the subject is at risk of having such (*e.g.,* the risk is significantly increased vis-à-vis a control subject or subject population). The term "prediction of no" diseases or conditions as taught herein as described herein in a subject may particularly mean that the subject has a 'negative' prediction of such, *i.e.,* that the subject's risk of having such is not significantly increased vis-à-vis a control subject or subject population.

The methods as taught herein may be adequately qualified as *in vitro* methods in that they apply one or more *in vitro* processing and/or analysis steps to a sample removed from the subject. The term *"in vitro"* generally denotes outside, or external to, a body, e.g., an animal or human body. One understands that the present methods may generally comprise an examination phase in which data is collected from and/or about the subject.

The term "subject" as used herein typically and preferably denotes humans, but may also encompass reference to non-human animals, preferably warm-blooded animals, more preferably vertebrates, even more preferably mammals, such as, e.g., non-human primates, rodents, canines, felines, equines, ovines, porcines, and the like. Particularly intended are subjects known or suspected to have IHR. Suitable subjects may include ones presenting to a physician for a screening for IHR and/or with symptoms and signs indicative of IHR.

Despite the practical advantages of using the methods of the invention for the detection of hypersensitivity to drugs over existing methods, the sensitivity of these methods have been realized and for this reason not exploited so far. For not all available small compounds small compound-specific IgE assays are available. In general specific IgE assays typically exhibit a varying accuracy depending on the compound and the clinical phenotype. Importantly, sensitization, as revealed by a positive compound-specific IgE result, frequently fails to correlate with clinical reactivity. Accordingly, to differentiate between an IHR and a sensitization reaction in a subject, the methods as taught herein may be performed after a subject has responded positively to an IgE test specific. Therefore, in particular embodiments, said subject is a subject that responded positive on IgE test.

The methods of the invention may be of interest to determining hypersensitivity both in patients which have and in patients which have not yet been exposed to the compound. In particular embodiments of the methods of the invention, said subject was exposed to said small compound or said drug prior to obtaining serum from said subject.

In particular embodiments, the method for diagnosing IHR is a method for differentiating between a genuine IHR or IgE/FcεRI-dependent allergy against a small compound or a drug and a sensitization to said small compound or said drug resulting in tolerance in a subject.

In more particular embodiments, said subject was exposed to said small compound or said drug from 0 minutes to 360 minutes, from 0 minutes to 300 minutes, from 0 minutes to 240 minutes, from 0 minutes to 180 minutes, from 0 minutes to 120 minutes, or from 0 minutes to 60 minutes prior to obtaining serum from said subject.

In particular embodiments, said subject is a subject having one or more of the symptoms selected from the group consisting of clinical urticaria, vomiting, wheezing, angioma, bronchospasm and/ anaphylaxis within a period of from 0 minutes to 360 minutes, from 0 minutes to 300 minutes, from 0 minutes to 240 minutes, from 0 minutes to 180 minutes, from 0 minutes to 120 minutes, or from 0 minutes to 60 minutes after having been administered the small molecule or drug.

In particular embodiments, the *in vitro* method as taught herein is a method for diagnosing an immediate hypersensitivity reaction (IHR) against a small compound in a subject, for diagnosing immediate hypersensitivity against a small compound in a subject and/or for predicting the risk of anaphylaxis upon re-exposure of a small compound to a subject.

The term "small compound" as used herein encompasses organic and inorganic compounds with a molecular weight of at most 9000 Da, at most 8000 Da, at most 7000 Da, at most 6000 Da, at most 5000 Da, e.g. up to about 4000, preferably up to 3000 Da, more preferably up to 2000 Da, even more preferably up to about 1000 Da, e.g., up to about 900, 800, 700, 600 or up to about 500 Da, preferably at most 600 Da. The term "small compound" encompasses small compounds to which a subject is exposed knowingly, for a specific purpose, for example, as a diagnostic or therapeutic compound, but also encompasses compounds to which a subject is exposed unknowingly, for example, as an adjuvant of a pharmaceutical composition or as a food-additive. Non-limiting examples of small compounds include therapeutic compounds, diagnostic compounds (e.g. contrast agents), adjuvants for therapeutic or diagnostic compositions, small compounds present in food products (e.g. pesticides, fungicides, metals) and small compounds present in cosmetics (e.g. additives to creams, toothpaste, deodorants, and antiperspirants). Accordingly, the small compound should not be considered to be limited to small molecules as known in the art. Non-limiting examples of small compounds include antiseptics (such as cationic polybiguanides like chlorhexidine), disinfectants, antibiotics, anticonvulsants, anaesthetics, neuromuscular blocking drugs (NMBDs), chemotherapeutic drugs and non-steroidal anti-inflammatory drugs (NSAIDs).

In preferred embodiments, the small compound is a chemical compound. In preferred embodiments, the small compound is not a biological macromolecule, such as a protein, peptide or polypeptide (e.g. antibody, food protein, pollen, venom) or a nucleic acid. In preferred embodiments, the small compound is not a biological compound.

In particular embodiment, the small compound is a hapten. The term "hapten" as used herein refers to a chemically reactive small molecule which is not immunogenic as such, but which is immunogenic when conjugated to a larger carrier, such as a protein. The carrier typically does not elicit an immune response by itself.

In particular embodiments, the small compound is a drug or a medicinal product. In other words, in particular embodiments, the small compound is a therapeutic or diagnostic compound or a small compound used for diagnostic and therapeutic purpose.

In particular embodiments, the *in vitro* method as taught herein is a method for diagnosing an immediate hypersensitivity reaction (IHR) against a drug in a subject, for diagnosing immediate hypersensitivity against a drug in a subject and/or for predicting the risk of anaphylaxis upon re-exposure of a drug to a subject.

The term "drug" or "medicinal product" as used herein any substance or combination of substances, such as chemical and/or biological substances or compounds, that produces a biological effect (e.g. a pharmacological, immunological or metabolic action) when administered to a living subject. More particularly, a drug or medicinal product may be administered to a subject to diagnose, cure, mitigate, treat or prevent of a disease or to restore, correct or modify physiological functions. The term "drug" is typically used in the United States to refer to such a substance, while the term "medicinal product" is typically used in Europe to refer to such a substance. The term "drug" or "medicinal product" as used herein should not be considered to be limited to small compounds but also encompasses biologicals that are either prepared from living organisms or derived from substances produced from living organism, such as, but not limited to, vaccines, cell or gene therapies, hormones, cytokines, tissue growth factors or monoclonal antibodies. Biologicals may have a molecular weight of from 5 to 200 kDa or from 5 to 150 kDa. Preferably, the term "drug" or "medicinal product" refers to the one or more active ingredients or substances present in a pharmaceutical composition.

In more particular embodiments, the drug or small compound is a therapeutic or diagnostic compound selected from the group consisting of anti-inflammatory drugs, analgesics (e.g. opioids and non-opioids), anesthetics, antibacterials (e.g. antibiotics), anticancer agents, anticonvulsants, antidementia agents, antidepressants, antidotes and antitoxins, antiemetics, antifungals, anti-inflammatory agents (e.g. corticosteroids and nonsteroidal anti-inflammatory drugs (NSAIDs)), antimigraine agents, antimyasthenic agents, antimycobacterials, antineoplastics, antiparasitics, antiparkinson agents, antipsychotics, antivirals (e.g. HIV antiretrovirals and direct-acting hepatitis C drugs), antiseptic, anxiolytic (anti-anxiety) agents, bipolar agents, blood glucose regulators (e.g. insulin and other diabetes medications), blood products (e.g. anticoagulants, antiplatelet drugs, fibrinolytic drugs), cardiovascular agents (e.g. beta-blockers and ACE inhibitors), central nervous system agents (e.g. psychiatric drugs, amphetamines), dental and oral agents, dermatological (skin) agents, disinfectant, enzyme replacement agent, gastrointestinal agents (e.g. H2 blockers and proton pump inhibitors), genitourinary (genital and urinary tract) agents, hormonal agents (e.g. adrenal, pituitary, prostaglandis, tyroid, sex hormones such as estrogen, testosterone, and anabolic steroids), hormonal suppressant (e.g. adrenal, parathyroid, pituitary, sex hormones, thyroid), immunological agents (e.g. vaccines and disease-modifying anti-rheumatic drugs (DMARDs)), inflammatory bowel disease agents, metabolic bone disease agents, ophthalmic (eye) agents, otic (ear) agents, respiratory tract agents (e.g. bronchodilators), sedatives and hypnotics and skeletal muscle relaxants.

The person skilled in the art will understand that the methods of determining IHR against compound of interest (being a drug and/or a small compound) as taught herein could also be performed by contacting the mast cells obtained in step (a) with a compound which is structural similar to the small compound of interest. Preferably, the structure of the structurally similar compound is substantially identical (i.e., largely but not wholly identical) to the structure of the compound of interest e.g., at least about 80% identical or at least about 85% identical, e.g., preferably at least about 90% identical, e.g., at least 91% identical, 92% identical, more preferably at least about 93% identical, e.g., at least 94% identical, even more preferably at least about 95% identical, e.g., at least 96% identical, yet more preferably at least about 97% identical, e.g., at least 98% identical, and most preferably at least 99% identical to the structure of the compound of interest. The structurally similar compound may encompass minor structural variations compared to the compound of interest, such as one or more substitutions.

Also, provided herein is a method of determining IHR against a small compound or a drug in a subject, comprising the steps of:
(a) contacting mast cells with serum obtained from said subject;
(b) contacting the mast cells obtained in step (a) with said small compound or said drug;
(c) detecting mast cell activation and/or degranulation of the cells obtained in step (b); and
(d) diagnosing IHR in the subject based on the detection of mast cell activation and/or degranulation.

In particular embodiments, said method is a method of diagnosis. In particular embodiments, said method is a method of treating (including preventing) a IHR against a small compound or a drug in a subject, wherein the method comprises determining whether or not the subject has immediate hypersensitivity against the small compound or the drug and administering to said subject in which said immediate hypersensitivity has been determined an effective amount of a therapeutic (including prophylactic) agent against IHR.

Accordingly, in particular embodiments, the method further comprises the step of (e) administering to said subject diagnosed with IHR a therapy for IHR, such as administering to said subject an effective amount of a therapeutic agent for IHR.

The terms "treat" or "treatment" encompass both the therapeutic treatment of an already developed disease or condition, such as the therapy of anaphylaxis, as well as prophylactic or preventive measures, wherein the aim is to prevent or lessen the chances of incidence of an undesired affliction, such as to prevent progression of IHR. Beneficial or desired clinical results may include, without limitation, alleviation of one or more symptoms or one or more biological markers, diminishment of extent of disease or condition, stabilised (i.e., not worsening) state of disease, delay or slowing of disease progression, amelioration or palliation of the disease state, and the like. "Treatment" can also mean prolonging survival as compared to expected survival if not receiving treatment.

Non-limiting examples of therapies for IHR include intramuscular administration or intravenous (IV) of adrenaline (epinephrine), oral or intravenous (IV) administration of antihistamine, oral or intravenous administration of cortisone, administration of oxygen, support of blood pressure with intravenous (IV) fluids, and surgical procedures such as tracheotomy, for example, if there is severe laryngeal edema.

The term "effective amount" as used herein may refer to a prophylactically effective amount, which is an amount of an active compound or pharmaceutical agent, more particularly a prophylactic agent, that inhibits or delays in a subject the onset of a disorder or condition as being sought by a researcher, veterinarian, medical doctor or other clinician, or may refer to a therapeutically effective amount, which is an amount of active compound or pharmaceutical agent, more particularly a therapeutic agent, that elicits the biological or medicinal response in a subject that is being sought by a researcher, veterinarian, medical doctor or other clinician, which may include *inter alia* alleviation of the symptoms of the disease or condition being treated. Methods are known in the art for determining therapeutically and prophylactically effective doses for the agents as disclosed herein.

The term "administration" or "administering" as used herein refers to the giving of a certain treatment of IHR to a subject in need of such a treatment. Such a treatment for IHR can be a therapeutic agent as described elsewhere herein. The route of administration may be essentially any route of administration, such as without limitation, oral administration (such as, *e.g.,* oral ingestion), intranasal administration (such as, e.g., intranasal inhalation or intranasal mucosal application), parenteral administration (such as, e.g., subcutaneous, intravenous, intramuscular, intraperitoneal or intrasternal injection or infusion), transdermal or transmucosal (such as, e.g., oral, sublingual, intranasal) administration, topical administration, rectal, vaginal or intra-tracheal instillation, and the like. In this way, the therapeutic effects attainable by the methods of the invention can be, for example, systemic, local, tissue-specific, etc., depending of the specific needs of a given application of the invention.

The term "mast cell", "mastocyte" or "labrocyte" as used herein refers to an immune cell of the myeloid lineage which contain many granules rich in histamine and heparin, which have a round nucleus and which are resident in tissues, such as connective tissue, throughout the body. Mast cells play a role in allergies, anaphylaxis, wound healing, angiogenesis and immune tolerance. Preferably, the mast cells are human mast cells. In particular embodiments, the mast cells are cells that have been shown to be particularly capable of activation and/or degranulation.

In particular embodiments, cells with a similar functionality than mast cells, i.e. capable of activation and/or degranulation upon exposure to a small compound or a drug, may be used instead of the mast cells. Non-limiting examples of such cells are cell lines, such as HEK293 cells, stably or transiently transfected with an IgE receptor.

In particular embodiments, the mast cells are a primary culture of mast cells or mast cell lines.

Non-limiting examples of mast cell lines include immortalized human mast cell line (LUVA), human mast cell line 1 (HMC-1), laboratory of allergic diseases 2 (LAD2) and ROSA^{KIT WT}.

In particular embodiments, the primary culture of mast cells are cultured donor mast cells (dMCs), preferably cultured human donor mast cells. The dMCs may be derived from bone marrow, peripheral blood or umbilical cord, preferably from peripheral blood. Methods for preparing primary cultures of mast cells are well known in the art, for example, as described in Cop N et al. Phenotypic and functional characterization of in vitro cultured human mast cells. Cytometry B Clin Cytom. 2017 Sep;92(5):348-354.

In particular embodiments, the cultured donor mast cells are obtained by
- isolating peripheral blood mononuclear cells from peripheral blood obtained from a healthy subject;
- isolating CD34+ progenitor cells from said peripheral blood mononuclear cells; and
- culturing said CD34+ positive cells during a period from 4 to 12 weeks, preferably from 4 to 8 weeks.

Preferably, the time of culture of the stem cells is as short as possible.

In particular embodiments, the donor mast cells are cultured for a period from 4 to 12 weeks, preferably from 4 to 8 weeks, prior to contacting said donor mast cells with serum obtained from said subject.

Because the characteristics of the mast cells used in the methods as taught herein are well known (e.g. their ability to be activated and/or to degranulate) prior to performing the methods as taught herein on said mast cells, the methods as taught herein are more reliable than, for example the BAT, in which viable cells need to be obtained from the subject to be diagnosed. Furthermore, the methods as taught herein could be repeated on different mast cells, such as on mast cells from a second donor, for example if there would be any doubt that the obtained diagnosis is correct.

Accordingly, in particular embodiments, the donor of the mast cells and the donor of the serum is not the same subject. In other words, the donor mast cells are allogeneic mast cells.

In particular embodiments, the donor mast cells are donor mast cells pooled from two or more different donors.

The term "serum" as used herein refers to the liquid fraction of whole blood isolated from a subject that is collected after the whole blood is allowed to clot. Serum typically does not comprise any white blood cells, red blood cells, platelets or clotting factors. Methods for preparing serum from whole blood are well known in the art and include centrifuging a whole blood sample at 1000 to 2000 g for 10 minutes after blood clotting and subsequently collecting the supernatant.

Unlike BAT, the methods as taught herein only make use of the serum of the patient and not of viable blood cells. Accordingly, the methods as taught herein are not affected by the deterioration of blood cells in the blood sample and hence, the sample obtained from the patient does not need to be a fresh sample. As a result thereof, the methods as taught herein can be transported to and performed at a central diagnostic laboratory and can be repeated if part of the patient's serum is properly stored. Therefore, in particular embodiments, the serum sample is a freshly isolated serum sample, a serum sample which has been stored for at most 7 days at a temperature of from 4 to 8°C, or a freshly thawed serum sample.

The term "contact" or "contacting" as used herein means bringing one or more first components (such as one or more molecules, biological entities, cells, or materials) together with one or more second components (such as one or more molecules, biological entities, cells, or materials) in such a manner that the first component(s) can - if capable thereof - bind or modulate the second component(s) or that the second component(s) can - if capable thereof - bind or modulate the first component(s). Such modulation may occur either directly, i.e., by way of direct interaction between the first and second component(s); or indirectly, e.g., when the first component(s) interact with or modulate one or more further component(s), one or more of which in turn interact with or modulate the second component(s), or vice versa. The term "contacting" may depending on the context be synonymous with "exposing", "incubating", "mixing", "reacting", "treating", or the like.

In particular embodiments, the step of contacting the mast cells with serum obtained from said subject is performed for a period of at least 30 minutes, at least 1 hour, at least 2 hours, at least 3 hours, at least 4 hours, at least 5 hours, at least 6 hours, at least 7 hours, at least 8 hours, at least 9 hours, at least 10 hours, at least 11 hours, or at least 12 hours, such as over night.

In particular embodiments, the step of contacting the mast cells with serum obtained from said subject is performed at a temperature of 35 to 40°C, preferably at a temperature of 37°C. For example, the step of contacting the mast cells with serum obtained from said subject can be performed in a humidified CO₂ incubator at 37°C.

In particular embodiments, the step of contacting the mast cells with serum obtained from said subject allows to passively sensitize the mast cells.

In particular embodiments, the culture medium in which the mast cells are cultured is removed from the mast cells prior to contacting the mast cells with serum obtained from said subject.

In particular embodiments, the serum from the subject is undiluted serum or diluted serum, such as a 1:2, 1:5 or 1:10 dilution, preferably a 1:2 dilution of the serum, for example 1 part of undiluted serum may be mixed with 1 part of mast cells in culture medium.

After the mast cells have been contacted with the serum from the subject, the mast cells are exposed to the small compound or the drug to activate the mast cells. Exposure of the mast cells to a buffer or a substance which is not capable of eliciting mast cell activation and/or degranulation can be used as a negative control. The amount of small compound or drug used in the methods as taught herein is dependent on the type of small compound or drug being tested as well as on the type of mast cells being used. Preferably, the amount of the small compound or drug is an amount which is not toxic for to the mast cells.

Accordingly, in particular embodiments, the mast cells are contacted with a non-toxic concentration of said small compound or said drug. Methods to determine whether a certain concentration of a small compound or a drug is toxic to a cell are well known in the art and include cell viability or cytotoxicity assays such as MTT assays and the plotting of a dose-response curve. In particular embodiments, the small compound or drug is dissolved in culture medium for mast cells or a buffer.

In particular embodiments, the step of contacting the mast cells with said small compound or said drug is performed for a period of at least 2 minutes, at least 5 minutes, at least 10 minutes, at least 15 minutes, at least 20 minutes or at least 30 minutes, preferably at least 20 minutes. The period of contact with the small compound or the drug is dependent on the type of small compound or the drug. It is known in the art how to optimize contact period for each small compound or drug and include plotting of dose-response curves.

In particular embodiments, the mast cells are contacted in parallel with a range of concentrations of the small compound or the drug. For example, the range of concentrations may consist of four different concentrations, wherein each subsequent concentration is a 100-fold of the previous concentration; such as 0.05, 5, 500 and 500 000 ng/ml.

In particular embodiments, prior to contacting the mast cells with the small compound or the drug and after contacting the mast cells with serum obtained from said subject, the serum obtained from said subject is removed from the mast cells. This may be achieved by centrifuging the cells, removing the supernatants and resolving the cell pellet in a buffer or culture medium.

The methods as taught herein may comprise further steps to increase the efficiency of the method. For example, such steps may include steps to make the mast cells more sensitive to the small compound.

In particular embodiments, prior to contacting the mast cells with said small compound or said drug and after contacting the mast cells with serum obtained from said subject, the mast cells are pre-incubated with one or more priming cytokines selected from the group consisting of IL-33, IL-6 and IL-4, preferably IL-33.

In particular embodiments, the mast cells are pre-incubated with from 10 ng/ml to 1000 ng/ml, from 50 ng/ml to 500 ng/ml, or from 50 ng/ml to 300 ng/ml of priming cytokines selected from the group consisting of IL-33, IL-6 and IL-4.

In particular embodiments, the mast cells are pre-incubated with IL-33 or IL-4 for a period of at least 2 minutes, at least 5 minutes, at least 10 minutes, at least 15 minutes, at least 20 minutes or at least 30 minutes, preferably at least 20 minutes.

In particular embodiments, the mast cells are pre-incubated with IL-6 for a period from 4 to 10 weeks, preferably for a period of from 4 to 8 weeks.

Preferably, prior to determining the mast cell activation and/or degranulation of the mast cells, the mast cell activation and/or degranulation is stopped. In particular embodiments, prior to detecting mast cell activation and/or degranulation of the mast cells and after contacting the mast cells with the small compound or the drug, the mast cells are placed at a temperature from -4 to 10°C and/or the supernatants is removed from the mast cells.

Mast cell activation and/or degranulation can be detected by detecting or measuring the quantity or expression level of one or more biomarkers of mast cell activation and/or degranulation. These biomarkers may be present intracellularly of the mast cell, on the cell surface of the mast cell or may be secreted and/or released by the mast cell. Biomarkers secreted and/or released by the mast cell can, for example, be detected in the culture medium of the mast cells. More particularly, biomarkers secreted and/or released by the mast cell can be detected in the supernatants obtained after centrifuging the mast cells after contacting the mast cells with said small compound or said drug. Non-limiting examples of biomarkers of mast cell activation and/or degranulation present on the cell surface of the mast cells include the CD63 antigen, the CD107a antigen, the CD152 antigen, and the CD203c antigen. Non-limiting examples of biomarkers of mast cell activation and/or degranulation which are secreted and/or released by the mast cells include histamine, beta-tryptase, alpha-protryptase, prostaglandin D2 (PGD2) and beta-hexosaminidase.

Non-limiting examples of biomarkers of mast cell activation include the CD203c antigen.

The person skilled in the art will understand that the mRNA encoding a peptide-, polypeptide- or protein-biomarker of mast cell activation and/or degranulation present within the mast cell or present on the cell surface of the mast cell can serve as a biomarker for mast cell activation and/or degranulation; as typically the gene expression is modulated if the peptide, polypeptide or protein levels are modulated. For example, the CD63 mRNA encoding the CD63 antigen may be used as a biomarker for mast cell activation and/or degranulation.

Furthermore, the person skilled in the art will also understand that biomarkers of mast cell activation and/or degranulation present on the cell surface of the mast cells may also serve as biomarkers of mast cell activation and/or degranulation present within the mast cell, as prior to their presentation to the cell surface, these biomarkers are present within the mast cell, for example in late endosomes, lysosomes and/or secretory vesicles.

Furthermore, the person skilled in the art will also understand that biomarkers of mast cell activation and/or degranulation secreted and/or released by the mast cells may also serve as biomarkers of mast cell activation and/or degranulation present within the mast cell, as secretion or release of said biomarkers by the mast cells will typically lead to a decrease of the quantity of said biomarker within the mast cell.

A molecule or analyte such as a marker, peptide, polypeptide, protein, or nucleic acid, is "detected" in a sample when the presence or absence and/or quantity of said molecule or analyte is detected or determined in the sample, preferably substantially to the exclusion of other molecules and analytes.

Depending on factors that can be evaluated and decided on by a skilled person, such as *inter alia* the type of a biomarker (e.g., peptide, polypeptide, protein, or nucleic acid), the type of a sample, the expected abundance of the biomarker in the sample, the type, robustness, sensitivity and/or specificity of the detection method used to detect the biomarker, etc., the biomarker may be measured directly in the sample, or the sample may be subjected to one or more processing steps aimed at achieving an adequate measurement of the biomarker.

By means of example, the sample may be subjected to one or more isolation or separation steps, aimed at whereby the biomarker is isolated from the sample or whereby a fraction of the sample is prepared which is enriched for the biomarker. For example, if the biomarker is a peptide, polypeptide, or protein, any known protein purification technique may be applied to the sample to isolate peptides, polypeptides, and proteins therefrom. If the biomarker is a nucleic acid, any known nucleic acid purification technique may be applied to the sample to isolate nucleic acids therefrom. Non-limiting examples of methods to purify peptides, polypeptides, proteins, or nucleic acids may include chromatography, preparative electrophoresis, centrifugation, precipitation, affinity purification, etc.

As used herein, the term "purified" with reference to markers, peptides, polypeptides, proteins, or nucleic acids does not require absolute purity. Instead, it denotes that such markers, peptides, polypeptides, proteins, or nucleic acids are in a discrete environment in which their abundance (conveniently expressed in terms of mass or weight or concentration) relative to other analytes is greater than in the biological sample. A discrete environment denotes a single medium, such as for example a single solution, gel, precipitate, lyophilisate, etc. Purified nucleic acids, proteins, polypeptides or peptides may be obtained by known methods including, for example, laboratory or recombinant synthesis, chromatography, preparative electrophoresis, centrifugation, precipitation, affinity purification, *etc.*

Purified markers, peptides, polypeptides, proteins, or nucleic acids may preferably constitute by weight ≥ 10%, more preferably ≥ 50%, such as ≥ 60%, yet more preferably ≥ 70%, such as ≥ 80%, and still more preferably ≥ 90%, such as ≥ 95%, ≥ 96%, ≥ 97%, ≥ 98%, ≥ 99% or even 100%, of the protein content of the discrete environment. Protein content may be determined, e.g., by the Lowry method (Lowry et al. 1951. J Biol Chem 193: 265), optionally as described by Hartree 1972 (Anal Biochem 48: 422-427). Purity of peptides, polypeptides, or proteins may be determined by SDS-PAGE under reducing or non-reducing conditions using Coomassie blue or, preferably, silver stain. Quantity of nucleic acids may be determined by measuring absorbance A260. Purity of nucleic acids may be determined by measuring absorbance A260/A280, or by agarose- or polyacrylamide-gel electrophoresis and ethidium bromide or similar staining.

Any existing, available or conventional separation, detection and quantification methods may be used herein to measure the presence or absence (e.g., readout being present vs. absent; or detectable amount vs. undetectable amount) and/or quantity (e.g., readout being an absolute or relative quantity, such as, for example, absolute or relative concentration) of markers, peptides, polypeptides, proteins, or nucleic acids in samples.

For example, such methods may include biochemical assay methods, immunoassay methods, mass spectrometry analysis methods, or chromatography methods, or combinations thereof. The method of detection may vary depending on the location of the biomarker with regard to the mast cell, i.e. being intracellularly of the mast cells, on the cell surface of the mast cells and/or being secreted by the mast cells.

The term "immunoassay" generally refers to methods known as such for detecting one or more molecules or analytes of interest in a sample, wherein specificity of an immunoassay for the molecule(s) or analyte(s) of interest is conferred by specific binding between a specific-binding agent, commonly but without limitation an antibody, and the molecule(s) or analyte(s) of interest. Immunoassay technologies include without limitation flow cytometry (FCM), immunohistochemistry (IHC), immunocytochemistry (ICC), immunoprecipitation (IP), direct enzyme-linked immunosorbent assay (ELISA), indirect ELISA, sandwich ELISA, competitive ELISA, multiplex ELISA, radioimmunoassay (RIA), ELISPOT technologies, Western Blotting (WB), immunofluorescence (IF), and other similar techniques known in the art.

Further peptide or polypeptide separation, identification or quantification methods may be used, optionally in conjunction with any of the above described analysis methods, for measuring biomarkers in the present disclosure. Such methods include, without limitation, chemical extraction partitioning, isoelectric focusing (IEF) including capillary isoelectric focusing (CIEF), capillary isotachophoresis (CITP), capillary electrochromatography (CEC), and the like, one-dimensional polyacrylamide gel electrophoresis (PAGE), two-dimensional polyacrylamide gel electrophoresis (2D-PAGE), capillary gel electrophoresis (CGE), capillary zone electrophoresis (CZE), micellar electrokinetic chromatography (MEKC), free flow electrophoresis (FFE), etc.

The level of biomarkers at the nucleic acid level, more particularly RNA level, e.g., at the level of hnRNA, pre-mRNA, mRNA, or cDNA, may be detected using standard quantitative RNA or cDNA measurement tools known in the art. Non-limiting examples include hybridisation-based analysis, microarray expression analysis, digital gene expression (DGE), RNA-in-situ hybridisation (RISH), Northern-blot analysis and the like; PCR, RT-PCR, RT-qPCR, end-point PCR, digital PCR or the like; supported oligonucleotide detection, pyrosequencing, polony cyclic sequencing by synthesis, simultaneous bi-directional sequencing, single-molecule sequencing, single molecule real time sequencing, true single molecule sequencing, hybridization-assisted nanopore sequencing, sequencing by synthesis, or the like.

The terms "quantity", "amount" and "level" are synonymous and generally well-understood in the art. The terms as used herein may particularly refer to an absolute quantification of a molecule or an analyte in a sample, or to a relative quantification of a molecule or analyte in a sample, i.e., relative to another value such as relative to a reference value as taught herein, or to a range of values indicating a base-line expression of the biomarker. These values or ranges can be obtained from a single patient or from a group of patients.

An absolute quantity of a molecule or analyte in a sample may be advantageously expressed as weight or as molar amount, or more commonly as a concentration, e.g., weight per volume or mol per volume.

A relative quantity of a molecule or analyte in a sample may be advantageously expressed as an increase or decrease or as a fold-increase or fold-decrease relative to said another value, such as relative to a reference value as taught herein. Performing a relative comparison between first and second parameters (e.g., first and second quantities) may but need not require first to determine the absolute values of said first and second parameters. For example, a measurement method can produce quantifiable readouts (such as, e.g., signal intensities) for said first and second parameters, wherein said readouts are a function of the value of said parameters, and wherein said readouts can be directly compared to produce a relative value for the first parameter vs. the second parameter, without the actual need first to convert the readouts to absolute values of the respective parameters.

The terms "quantity" and "expression level" of said biomarker for mast cell activation and/or degranulation are used interchangeably in this specification to refer to the absolute and/or relative quantification, concentration level or amount of any such product in a sample.

In particular embodiments, the step of detecting mast cell activation and/or degranulation comprises detecting or measuring the quantity or expression level of one or more biomarkers of mast activation and/or degranulation in or on said mast cells or secreted by said mast cells.

In particular embodiments, the step of detecting mast cell activation and/or degranulation comprises detecting or measuring the quantity or expression level of one or more biomarkers selected from the group consisting of the CD63 antigen, the CD107a antigen, the CD152 antigen, the CD203c antigen, histamine, beta-tryptase, alpha-protryptase, PGD2 and beta-hexosaminidase.

In particular embodiments, the step of detecting mast cell activation and/or degranulation comprises detecting or measuring the quantity or expression level of the CD63 antigen, the CD107a antigen, the CD 152 antigen and the CD203c antigen, preferably the CD63 antigen, on the cell surface of said mast cells, preferably by flow cytometry. Methods for performing flow cytometry are well known in the art. For example, after contacting the mast cells with said small compound or said drug the mast cells may be stained with a labeled monoclonal antibody to said biomarker on the cell surface of said mast cells and subsequently the labelled mast cells may be sorted based on the detection of the labelled monoclonal antibody using a flow cytometer.

In particular embodiments, the step of attributing the detection or no detection of mast cell activation and/or degranulation to a particular diagnosis of IHR against said small compound or said drug, a particular diagnosis of immediate hypersensitivity against said small compound or said drug, and/or to a particular prediction of anaphylaxis upon re-exposure of a small compound or a drug to a subject comprises attributing the detection of mast cell activation and/or degranulation to the diagnosis of IHR against said small compound or daid drug, to the diagnosis of immediate hypersensitivity against said small compound or said drug, and/or to the prediction of anaphylaxis upon re-exposure of said small compound or said drug to a subject.

In particular embodiments, the step of detecting mast cell activation and/or degranulation comprises detecting the percentage of the mast cells in or on which one or more biomarkers of mast activation and/or degranulation are present.

The mast cell activation and/or degranulation of the mast cells as detected in step (c) can be compared to the mast cell activation and/or degranulation which occurs for a reference situation (e.g. with serum from a reference subject or with a reference small compound, a reference drugor buffer).

For example, as a reference, the mast cells can be contacted in step (b) with a buffer, substance, compound or drug known not to elicit mast cell activation and/or degranulation or known to elicit mast cell activation and/or degranulation in said subject.

For example, as a reference, the mast cells can be contacted in step (a) with serum from a subject known not to show IHR against said small compound or said drug or known to show IHR against said small compound or said drug.

The invention further provides an *in vitro* method for diagnosing IHR against a small compound or a drug in a subject, for diagnosing immediate hypersensitivity against a small compound or a drug in a subject and/or for predicting anaphylaxis upon re-exposure of a small compound or a drug to a subject, comprising the steps of
(a) contacting mast cells with serum obtained from said subject;
(b) contacting the mast cells obtained in step (a) with said small compound or said drug;
(c) detecting mast cell activation and/or degranulation of the cells obtained in step (b);
(d1) comparing the degree of mast cell activation and/or degranulation as determined in step (c) with a reference value or a threshold value, said reference value or threshold value representing a known diagnosis of IHR;
(d2) finding a deviation or no deviation between the degree of mast cell activation and/or degranulation as determined in step (c) and said reference value or said threshold value; and
(d3) attributing said finding of deviation or no deviation to a particular diagnosis of IHR against said small compound or said drug, a particular diagnosis of immediate hypersensitivity against said small compound or said drug and/or to a particular prediction of anaphylaxis upon re-exposure of said small compound or said drug to a subject.

Distinct reference values may represent the diagnosis of IHR *vs.* the absence of IHR (such as, e.g., healthy or recovered from IHR). In another example, distinct reference values may represent the diagnosis of IHR varying severity.

Alternatively, distinct reference values may represent the prediction of a risk (e.g., an abnormally elevated risk) of developing anaphylaxis upon re-exposure of a small compound to the subject *vs*. the prediction of no or normal risk of developing anaphylaxis upon re-exposure of a small compound to the subject.

Such comparison may generally include any means to determine the presence or absence of at least one difference and optionally of the size of such difference between values or profiles being compared. A comparison may include a visual inspection, an arithmetical or statistical comparison of measurements. Such statistical comparisons include, but are not limited to, applying an algorithm. If the values or biomarker profiles comprise at least one standard, the comparison to determine a difference in said values or biomarker profiles may also include measurements of these standards, such that measurements of the biomarker are correlated to measurements of the internal standards.

Reference values for the quantity or expression level of said one or more biomarkers of mast cell activation and/or degranulation may be established according to known procedures previously employed for other biomarkers. For example, a reference value of the amount of said one or more biomarkers of mast cell activation and/or degranulation for a particular diagnosis and/or prediction of a proliferative disease as taught herein may be established by determining the quantity or expression level of said one or more biomarkers of mast cell activation and/or degranulation in sample(s) from one individual or from a population of individuals characterised by said particular diagnosis and/or prediction of said disease or condition. Such population may comprise without limitation ≥ 2, ≥ 10, ≥ 100, or even several hundred individuals or more.

Hence, by means of an illustrative example, reference values of the quantity or expression level of said one or more biomarkers of mast cell activation and/or degranulation for the diagnosis of IHR *vs.* no such disease or condition may be established by determining the quantity or expression level of said one or more biomarkers of mast cell activation and/or degranulation in sample(s) from one individual or from a population of individuals diagnosed (e.g., based on other adequately conclusive means, such as, for example, clinical signs and symptoms, etc.) as, respectively, having or not having IHR.

In an embodiment, reference value(s) as intended herein may convey absolute quantities of the biomarker as intended herein. In another embodiment, the quantity of the biomarker in a sample from a tested subject may be determined directly relative to the reference value (e.g., in terms of increase or decrease, or fold-increase or fold-decrease). Advantageously, this may allow the comparison of the quantity or expression level of the biomarker in the sample from the subject with the reference value (in other words to measure the relative quantity of the biomarker in the sample from the subject vis-à-vis the reference value) without the need first to determine the respective absolute quantities of the biomarker.

As explained, the present methods may involve finding a deviation or no deviation between the quantity or expression level of said one or more biomarkers of mast cell activation and/or degranulation as described herein measured in a sample from a subject and a given reference value.

A "deviation" of a first value from a second value may generally encompass any direction (*e.g.,* increase: first value > second value; or decrease: first value < second value) and any extent of alteration.

For example, a deviation may encompass a decrease in a first value by, without limitation, at least about 10% (about 0.9-fold or less), or by at least about 20% (about 0.8-fold or less), or by at least about 30% (about 0.7-fold or less), or by at least about 40% (about 0.6-fold or less), or by at least about 50% (about 0.5-fold or less), or by at least about 60% (about 0.4-fold or less), or by at least about 70% (about 0.3-fold or less), or by at least about 80% (about 0.2-fold or less), or by at least about 90% (about 0.1-fold or less), relative to a second value with which a comparison is being made.

For example, a deviation may encompass an increase of a first value by, without limitation, at least about 10% (about 1.1-fold or more), or by at least about 20% (about 1.2-fold or more), or by at least about 30% (about 1.3-fold or more), or by at least about 40% (about 1.4-fold or more), or by at least about 50% (about 1.5-fold or more), or by at least about 60% (about 1.6-fold or more), or by at least about 70% (about 1.7-fold or more), or by at least about 80% (about 1.8-fold or more), or by at least about 90% (about 1.9-fold or more), or by at least about 100% (about 2-fold or more), or by at least about 150% (about 2.5-fold or more), or by at least about 200% (about 3-fold or more), or by at least about 500% (about 6-fold or more), or by at least about 700% (about 8-fold or more), or like, relative to a second value with which a comparison is being made.

Preferably, a deviation may refer to a statistically significant observed alteration. For example, a deviation may refer to an observed alteration which falls outside of error margins of reference values in a given population (as expressed, for example, by standard deviation or standard error, or by a predetermined multiple thereof, e.g., ±1xSD or ±2xSD or ±3xSD, or ±1xSE or ±2xSE or ±3xSE). Deviation may also refer to a value falling outside of a reference range defined by values in a given population (for example, outside of a range which comprises ≥40%, ≥ 50%, ≥60%, ≥70%, ≥75% or ≥80% or ≥85% or ≥90% or ≥95% or even ≥100% of values in said population).

In a further embodiment, a deviation may be concluded if an observed alteration is beyond a given threshold or cut-off. Such threshold or cut-off may be selected as generally known in the art to provide for a chosen sensitivity and/or specificity of the prediction methods, e.g., sensitivity and/or specificity of at least 50%, or at least 60%, or at least 70%, or at least 80%, or at least 85%, or at least 90%, or at least 95%.

For example, receiver-operating characteristic (ROC) curve analysis can be used to select an optimal cut-off value of the quantity of a given biomarker for clinical use of the present diagnostic tests, based on acceptable sensitivity and specificity, or related performance measures which are well-known *per se,* such as positive predictive value (PPV), negative predictive value (NPV), positive likelihood ratio (LR+), negative likelihood ratio (LR-), Youden index, or similar.

The present methods, uses, or products may further involve attributing any finding of a deviation or no deviation between the quantity or expression level of said one or more biomarkers of mast cell activation and/or degranulation measured in a sample from a subject and a given reference value to the presence or absence of IHR, a particular prediction of a risk for developing anaphylaxis or for developing anaphylaxis upon re-exposure of said small compound or said drug to the subject.

In the methods provided herein the observation of a deviation between the quantity or expression level of said one or more biomarkers of mast cell activation and/or degranulation in, on or secreted by the mast cells and a reference value can lead to the conclusion that the diagnosis and/or prediction of IHR in said subject is different from that represented by said reference value. Similarly, when no deviation is found between the quantity or expression level of one or more biomarkers of mast cell activation and/or degranulation in the mast cells and a reference value, the absence of such deviation can lead to the conclusion that the diagnosis and/or prediction of IHR in said subject is substantially the same as that represented by said reference value.

The person skilled in the art will understand that the deviation (e.g. increase or decrease) as well as the extend of the alteration may differ depending on the biomarker of mast cell activation and/or degranulation being used to detect mast cell activation and/or degranulation, as well as on the reference value or threshold value being used.

In particular embodiments, if the biomarker for mast cell activation and/or degranulation is the CD63 antigen, an increase in the quantity or expression level of CD63 antigen on the cell surface of the mast cells compared to a control (e.g. the quantity or expression level of CD63 antigen on the cell surface of the mast cells contacted with serum from a healthy subject) indicates the detection of mast cell activation and/or degranulation and the detection of mast cell activation and/or degranulation can be attributed to the diagnosis of IHR.

In particular embodiments, if the biomarker for mast cell activation and/or degranulation is the CD107a antigen, an increase in the quantity or expression level of CD107a antigen on the cell surface of the mast cells compared to a control (e.g. the quantity or expression level of CD107a antigen on the cell surface of the mast cells contacted with serum from a healthy subject) indicates the detection of mast cell activation and/or degranulation and the detection of mast cell activation and/or degranulation can be attributed to the diagnosis of IHR.

In particular embodiments, if the biomarker for mast cell activation and/or degranulation is the CD 152 antigen, an increase in the quantity or expression level of CD 152 antigen on the cell surface of the mast cells compared to a control (e.g. the quantity or expression level of CD152 antigen on the cell surface of the mast cells contacted with serum from a healthy subject) indicates the detection of mast cell activation and/or degranulation and the detection of mast cell activation and/or degranulation can be attributed to the diagnosis of IHR.

In particular embodiments, if the biomarker for mast cell activation and/or degranulation is the CD203c antigen, an increase in the quantity or expression level of CD203c antigen on the cell surface of the mast cells compared to a control (e.g. the quantity or expression level of CD203c antigen on the cell surface of the mast cells contacted with serum from a healthy subject) indicates the detection of mast cell activation and the detection of mast cell activation and/or degranulation can be attributed to the diagnosis of IHR.

In particular embodiments, if the biomarker for mast cell activation and/or degranulation is the histamine, beta-tryptase, alpha-protryptase, PGD2 and/or beta-hexosaminidase, an increase in the quantity or expression level of histamine, beta-tryptase, alpha-protryptase, PGD2 and/or beta-hexosaminidase in the supernatants of the mast cells compared to a control (e.g. the quantity or expression level of histamine, beta-tryptase, alpha-protryptase, PGD2 and/or beta-hexosaminidase in the supernatants of the mast cells of the mast cells contacted with serum from a healthy subject) indicates the detection of mast cell activation and/or degranulation and the detection of mast cell activation and/or degranulation can be attributed to the diagnosis of IHR.

While the invention has been described in conjunction with specific embodiments thereof, it is evident that many alternatives, modifications, and variations will be apparent to those skilled in the art in light of the foregoing description. Accordingly, it is intended to embrace all such alternatives, modifications, and variations as follows in the spirit and broad scope of the appended claims.

The above aspects and embodiments are further supported by the following non-limiting examples.

### EXAMPLES

### Example 1: use of the method as taught herein to diagnose Immediate hypersensitivity reaction (IHR) to chlorhexidine (CHX)

### 1. Materials and methods

### In vitro culture of human MCs

Human MCs were cultured as described in Cop N, et al. Influence of IL-6, IL-33, and TNF-alpha on human mast cell activation: Lessons from single cell analysis by flow cytometry. Cytometry B Clin Cytom 2018; 94:405-11. Briefly, peripheral blood mononuclear cells were isolated from 50 mL peripheral blood from healthy volunteers. CD34⁺ progenitor cells were enriched using the EasySep Human CD34 Selection Kit (Stemcell Technologies, Vancouver, Canada) according to the manufacturer's instructions. Isolated CD34⁺ progenitor cells were cultured in a serum-free methylcellulose-based medium (MethoCult SF H4236, Stemcell Technologies) supplemented with penicillin (100 units/mL, Life Technologies, Waltham, USA), streptomycin (100 µg/mL, Life Technologies), low-density lipoprotein (LDL, 10 µg/mL, Stemcell Technologies), 2-mercaptoethanol (55 µmol/L, Life Technologies), stem cell factor (SCF, 100 ng/mL, Miltenyi Biotec, Bergisch Gladbach, Germany), interleukin-3 (IL-3, 100 ng/mL, PeproTech, Rocky Hill, USA) and interleukin-6 (IL-6, 50 ng/mL, Miltenyi Biotec) during 4-5 weeks. Participants gave a written informed consent as approved by the Ethical Committee of the University Hospital of Antwerp (Belgium B300201316408).

### Sera from patients with perioperative anaphylaxis

Sera from 10 patients with a witnessed perioperative anaphylaxis and specific IgE (sIgE) to chlorhexidine (CHX) > 0.35 kUA/L (ImmunoCAP FEIA (Phadia Thermo Fisher scientific, Uppsala, Sweden), were selected. In five of these patients positive skin test and positive basophil activation test (BAT) confirmed the diagnosis of an IgE-mediated CHX hypersensitivity as described in Ebo DG, et al. IgE-mediated anaphylaxis from chlorhexidine: diagnostic possibilities. Contact Dermatitis 2006; 55:301-2. In the remainder five patients both skin test and BAT to the antiseptic was negative, indicating CHX serology likely to be clinically irrelevant. Demographic data and IgE results are displayed in table 1.

**TABLE 1: Demographics and results of confirmatory testing**

| **Patient** | **Sex** | **Age (y)** | **Total IgE** | **sIgE** | **ST** | **BAT** | **NAP6** | **Signs** |
|---|---|---|---|---|---|---|---|---|
| 1 | m | 41 | 68 | 10.3 | + | + | 2 | B, SK |
| 2 | m | 68 | 65 | 1.2 | + | + | 4 | H, TC |
| 3 | m | 58 | 60 | 8.77 | + | + | 4 | H, A, SK, MC |
| 4 | m | 73 | 149 | 0.66 | + | + | 4 | B, H, TC, SK |
| 5 | m | 64 | 195 | 3.28 | + | + | 3 | H, TC, B, A, SK, MC |
| 6 | m | 78 | 4848 | 1.71 | - | - | 2 | B, SK, MC |
| 7 | v | 54 | 815 | 6.8 | - | - | 4 | H, TC |
| 8 | v | 51 | 188 | 3.6 | - | - | 3 | H, TC, SK, MC |
| 9 | m | 64 | 6079 | 24.8 | - | - | 4 | S |
| 10 | v | 44 | 2483 | 2.17 | - | - | 4 | H, B |

M, male; f, female; sIgE, specific IgE chlorhexidine; ST, skin test; BAT, basophil activation test; +, positive; -, negative; H, hypotension; TC, tachycardia; A, angio-edema; B, Bronchospasm; S, Shock; MC, mucocutane lesions; SK, skin lesions.

### Activation

Degranulation of dMC was measured by overnight passively sensitizing the cells with serum at 37°C in a humidified CO₂-incubator. Next, dMC^{IgE+} were centrifuged (500g, 5 minutes, 20°C) and the cell pellet was resolved in pre-warmed Tyrode's buffer (Sigma-Aldrich, St. Louis, USA) at a concentration of 0.5x10⁶ cells/mL. Thereafter, 100 µL of the cells were pre-incubated with IL-33 (100 ng/mL) (Peprotech) for 20 minutes on 37°C. Subsequently, these pre-incubated dMC^{IgE+} were stimulated with 100 µL Tyrode's buffer as a negative control or 100 µL of CHX (Sigma-Aldrich) during 20 minutes at 37°C. The final concentrations of CHX were: 0.05, 5, 500, 50 000 ng/mL. Reactions were stopped by placing the cells on ice and removing the supernatants (500 g, 5 minutes, 4°C). Cells were stained with monoclonal anti-human CD117-APC (clone 104D2, BD Biosciences, Erembodegem, Belgium), CD203c-PeCy7 (clone NP4D6, eBioscience) and CD63-FITC (clone H5C6, BD Bioscience) for 20 minutes at 4°C. Finally, cells were washed and resolved in PBS with 0.1% sodium azide and measured. Degranulation of dMCs was measured as surface upregulation of the lysosomal degranulation marker CD63. The mast cell activation test is repeated on dMCs of two different volunteers.

### Flow cytometric analysis

Flow cytometric analysis was performed on a FACSCanto II flow cytometer (BD Immunocytometry Systems, San Jose, CA) equipped with three lasers (405 nm, 488 nm and 633 nm). Correct compensation settings for antibodies conjugated with fluorochromes were performed using BD CompBeads (BD Biosciences). Flow cytometric data were analyzed using Kaluza Analysis 1.5 software (Beckman Coulter, USA). Unstained samples were used to set a marker between positive and negative cells according to the 99th percentile. A fluorescence minus one (FMO) was used to set a marker between positive and negative cells. Mast cells were gated out as CD117 and CD203c positive cells. Results are expressed as mean and range.

### 2. Results

A representative individual plot is shown in figure 1. In resting dMC there was no expression of CD63. As shown in figure 2, unlike dMC^{IgE-}, in dMC^{IgE+}, that is cells, passively sensitized with patient's sera, CD63 was upregulated after activation with CHX, for 1% (1-22), 10% (1-76), 57% (6-86), 31% (0-88) for the corresponding concentrations of 0.05, 5, 500, 50000 ng/mL CHX. However, degranulation of dMC^{IgE+} was restricted to the five patients who demonstrated a positive skin test and BAT to CHX. In patients with an isolated sIgE CHX no upregulation of CD63 was demonstrable. As shown in panel B of figure 2, similar observation were done with MC from a second donor, adding rigor to our analyses. The overlook the effect of the dMC, we repeated these experiment on the cells of a different donor. Similar results of CD63 upregulation is obtained with the second donor 2% (0-21), 7% (2-44), 30% (3-64) and 45% (0-73) for the corresponding concentrations. (figure 2). Note that data from dMC^{IgE-} are not shown.

### 3. Discussion

Chlorhexidine (1:6-di(4-chlorophenyldiguanido)-hexane) is a synthetic cationic bis-biguanide with two biguanide groups both linked to a terminal 4-chlorophenyl group with the resultant chloroguanide structures connected via hexamethylene bridge. CHX, usually a gluconate or acetate salt, has a widespread application in various domestically and industrially products as well as in the medical environment. Today, CHX has evolved to one of the principal causes of perioperative anaphylaxis and different efforts have been undertaken to identify the fine structural specificities of the CHX epitopes complementary to CHX-reactive sIgE antibodies . In clinical practise diagnosis of IgE/FcεRI-dependent CHX allergy generally rests upon an evocative story combined with two or more positive tests, that is, sIgE, skin testing and a mediator release test or BAT. Present inventors selected the sera of patients with an evocative history displaying combined positive results for sIgE but also skin testing and a CD63-based BAT. In a separate experiment sera of patients with an isolated positive sIgE result were selected to study whether the technique benefits unravelling significance of sensitization as reflected by serologic findings. Present experiments show that dMC can effectively be loaded with CHX-reactive sIgE antibodies from patients with positive skin tests and CD63-based BAT and that these dMC^{IgE+} can subsequently be triggered to degranulate in response toCHX. Moreover, the method for diagnosing as taught herein reveals to be sensitive as successful passive sensitisation can be attained for titres of CHX-reactive sIgE as low as 0.66 kUA/L in the traditional ImmunoCAP assay. In contrast, when dMC, from the same donor, are loaded with CHX-reactive sIgE antibodies obtained from patients with negative skin test and CD63-based results, cells remain unresponsive to the antiseptic. In other words, the method for diagnosing as taught herein enables to discriminate between CHX allergy and CHX sensitization, as revealed by an isolated positive drug-sIgE result. Although, in the absence of a CHX challenge test, no absolute conclusions can be drawn, present findings militate against the practice to use sIgE antibodies to CHX in isolation to confirm IgE/FcεRI CHX allergy, especially when total IgE is elevated. To avoid misdiagnosis, the sIgE assay should be complemented with skin tests, BAT and/or the method for diagnosing as taught herein, especially in perioperative hypersensitivity as an erroneous diagnosis of CHX allergy entails a risk for overlooking the genuine culprit. Consistent with the general belief that IDH to CHX is IgE/FcεRI-dependent, present inventors could not elicit activation/degranulation of unloaded cells indicating an alternative MC activation pathway such as off target occupation of the MRGPRX2 receptor.

In conclusion, we demonstrate that application of the MAT extends beyond allergies towards proteinaceous allergens. Actually, it is shown that the technique can be used to diagnose IgE/FcεRI-dependent allergy to small compounds including drugs with high analytical sensitivity. Besides, the method for diagnosing as taught herein can be applied to assess the clinical relevance of drug-sIgE antibodies in their ability to elicit MC degranulate and therefore discriminate between allergy and merely sensitization as reflected by a positive sIgE result. Finally, the method for diagnosing as taught herein can facilitate investigation of the underlying mechanisms that govern and culminate in MC activation/degranulation in response to drugs. This is because the method for diagnosing as taught herein, unlike basophil activation experiments, allows simulatenous exploration of IgE/FcεRI- and MRGPRX2-dependent effector cell activation/degranulation.

## Claims

1. An *in vitro* method for diagnosing an immediate hypersensitivity reaction (IHR) against a small compound or a drug in a subject, comprising the steps of
- (a) contacting mast cells with serum obtained from said subject;
- (b) contacting the mast cells obtained in step (a) with said small compound or said drug;
- (c) detecting mast cell activation and/or degranulation of the mast cells obtained in step (b);
- (d) attributing the detection or no detection of mast cell activation and/or degranulation to a particular diagnosis of IHR.

2. The method according to claim 1, wherein said method is a method for diagnosing IHR against a small compound and step (b) comprises contacting the mast cells obtained in step (a) with said small compound.

3. The method according to claim 1, wherein said method is a method for diagnosing IHR against a drug and step (b) comprises contacting the mast cells obtained in step (a) with said drug.

4. The method according to claim 1 or 2, wherein said small compound has a molecular weight of at most 9000 Da, preferably at most 1000 Da.

5. The method according to any one of claims 1, 2 or 4, wherein said small compound is a diagnostic or a therapeutic compound.

6. The method according to any one of claims 1 to 5, wherein in step (b) the mast cells are contacted with a non-toxic concentration of said small compound or said drug.

7. The method according to any one of claims 1 to 6, wherein said mast cells used in step (a) are cultured donor mast cells (dMCs).

8. The method according to claim 6, wherein said dMCs used in step (a) are obtained by
- isolating peripheral blood mononuclear cells from peripheral blood obtained from a healthy subject;
- isolating CD34+ progenitor cells from said peripheral blood mononuclear cells; and
- culturing said CD34+ positive cells during a period from 4 to 12 weeks.

9. The method according to any one of claims 1 to 8, wherein mast cell activation and/or degranulation in step (c) is detected by detecting one or more biomarkers of mast activation and/or degranulation in or on said mast cells or secreted by said mast cells.

10. The method according to claim 9, wherein mast cell degranulation in step (c) is detected by determining overexpression of the CD63 antigen on the cell surface of said mast cells.

11. The method according to any one of claims 1 to 10, further comprising a step of pre-incubating said mast cells as obtained in step (a) with one or more priming cytokines selected from the group consisting of IL-33, IL-6 and IL-4, prior to step (b).
